(19)

Europäisches
Patentamt
European
Patent Office
Office européen
des brevets

(11) **EP 3 438 269 A1**

(12) **EUROPEAN PATENT APPLICATION**
published in accordance with Art. 153(4) EPC

(43) Date of publication:
**06.02.2019 Bulletin 2019/06**

(21) Application number: **17775089.0**

(22) Date of filing: **28.03.2017**

(51) Int Cl.:
***C12P 7/10*** *(2006.01)*　　　***C12P 19/14*** *(2006.01)*
***C13K 1/02*** *(2006.01)*

(86) International application number:
**PCT/JP2017/012643**

(87) International publication number:
**WO 2017/170552 (05.10.2017 Gazette 2017/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.03.2016 JP 2016066886**

(71) Applicant: **Toray Industries, Inc.**
**Tokyo 103-8666 (JP)**

(72) Inventors:
- **MINAMINO Atsushi**
  **Kamakura-shi**
  **Kanagawa 248-8555 (JP)**

- **FUNADA Shigeyuki**
  **Kamakura-shi**
  **Kanagawa 248-8555 (JP)**
- **KURIHARA Hiroyuki**
  **Kamakura-shi**
  **Kanagawa 248-8555 (JP)**
- **ASAHI Yuka**
  **Kamakura-shi**
  **Kanagawa 248-8555 (JP)**
- **KASAHARA Takuya**
  **Kamakura-shi**
  **Kanagawa 248-8555 (JP)**
- **YAMADA Katsushige**
  **Kamakura-shi**
  **Kanagawa 248-8555 (JP)**

(74) Representative: **Kador & Partner PartG mbB**
**Corneliusstraße 15**
**80469 München (DE)**

(54) **METHOD FOR PRODUCING SUGAR LIQUOR**

(57)　The present invention relates to a method of efficiently producing a sugar liquid having a high quality from a cellulose-containing biomass. More specifically, the present invention relates to a method of producing a sugar liquid, the method including: the step of obtaining an alkaline filtrate by allowing an alkaline aqueous medium to pass through a cellulose-containing biomass; the recirculation filtration step of allowing the alkaline filtrate to repeatedly pass through the cellulose-containing biomass to produce a cellulose-containing solid component; and the step of hydrolyzing the cellulose-containing solid component to obtain the sugar liquid.

EP 3 438 269 A1

**Description**

CROSS-REFERENCE TO RELATED APPLICATIONS

The present patent application is based upon and claims the benefit of priority from previously filed Japanese Patent Application No. 2016-066886 (filed on March 29, 2016), the entire disclosure of which is incorporated herein by reference.

BACKGROUND OF THE INVENTION

Field of the Invention

The present invention relates to a method of producing a sugar liquid which can be used as a fermentation raw material or the like, from a cellulose-containing biomass.

Background Art

Fermentation production processes for producing chemicals using saccharides as raw materials are employed in the production of various types of industrial raw materials. Currently, saccharides derived from edible raw materials such as sugarcane, starches and sugar beets are industrially used as saccharides for use as fermentation raw materials. However, in view of an increase in the cost of edible raw materials due to an increase in the world population in the future, or from an ethical point of view that the industrial use of edible raw materials may compete with the use thereof as food, it is a future challenge to develop a process for more efficiently producing a sugar liquid from a renewable, non-edible resource, namely, a cellulose-containing biomass, or a process for efficiently converting the resulting sugar liquid as a fermentation raw material into an industrial raw material.

Saccharides contained in cellulose-containing biomass raw materials are embedded within cell walls having a complicated structure. Therefore, a technique is known to subject a biomass raw material to an alkaline treatment before carrying out an enzymatic hydrolysis, so that the enzyme works efficiently.

For example, in order to improve the rate of enzymatic hydrolysis of cellulose, a technique is disclosed in which: a cellulose-containing product is subjected to an alkaline treatment by being brought into contact with an aqueous alkaline solution; the treated cellulose-containing product is washed with water and/or an aqueous acidic solution; and then an enzyme treatment is carried out by bringing the cellulose-containing product into contact with an aqueous solution containing a cellulolytic enzyme and a pH buffer, within the range of buffer solution concentration of from 0 to 250 mM (Patent Document 1).

Further, a pretreatment method for carrying out an enzyme treatment of a biomass is disclosed, which method is characterized in that the biomass is supplied into a twin screw extruder; an aqueous solution of an alkaline compound is injected into the extruder while supplying the biomass; kneading the biomass and the aqueous solution in the extruder to allow a reaction to proceed (Patent Document 2).

In order to reduce the cost of producing sugars from a biomass, a method is disclosed in which; an herbaceous biomass or woody biomass is subjected to an alkaline treatment using an alkaline solution; solid-liquid separation is carried out to separate the resulting alkaline treated solution into an alkaline solution and a solid component; the separated alkaline solution is supplemented with an alkaline substance, to be recycled to the alkaline treatment step (Patent Document 3).

Further, in order to improve the efficiency of enzymatic saccharification and to drastically reduce the amount of neutralization effluent, an enzymatic saccharification method of a cellulose-based biomass raw material is disclosed, which method is characterized in that: a slurry containing a cellulose-based biomass raw material which has been cut, crushed, ground, mashed or powdered, calcium hydroxide, and water is prepared to carry out an alkaline treatment of the raw material, followed by solid-liquid separation; solids obtained by the solid-liquid separation, or a mixture of the solids and water is neutralized using carbon dioxide to be adjusted to a pH within the range of from 5 to 8; and an enzymatic saccharification reaction is carried out (Patent Document 4).

However, a method of efficiently obtaining a sugar liquid having a high quality from a cellulose-containing biomass is still demanded.

Prior Art Documents

Patent Documents

Patent Document 1: JP 2011-135861 A
Patent Document 2: JP 59-192094 A
Patent Document 3: JP 2014-23484 A
Patent Document 4: JP 2013-220067 A

SUMMARY OF THE INVENTION

An object of the present invention is to provide a method of efficiently producing a sugar liquid from a cellulose-containing biomass. Another object of the present invention is to provide a method of producing a sugar liquid having a high quality from a cellulose-containing biomass.

The present inventors have found out that it is possible to efficiently obtain a sugar liquid by subjecting a cellulose-containing biomass to a specific pretreatment in which an alkaline filtrate is repeatedly used. Further, the present inventors have found out that the sugar liquid obtained as described above contains sugars at a high-purity. The present invention has been made based on such findings.

The present invention encompasses the following [1] to [16].

[1] A method of producing a sugar liquid, the method including:

the step of obtaining an alkaline filtrate by allowing an alkaline aqueous medium to pass through a cellulose-containing biomass;
the recirculation filtration step of allowing the alkaline filtrate to repeatedly pass through the cellulose-containing biomass to produce a cellulose-containing solid component; and
the step of hydrolyzing the cellulose-containing solid component to obtain the sugar liquid.

[2] The method of producing a sugar liquid according to [1], wherein the step of obtaining the alkaline filtrate includes supplying the cellulose-containing biomass and the alkaline aqueous medium to a filtration apparatus, and allowing the alkaline aqueous medium to pass through the cellulose-containing biomass using the filtration apparatus.

[3] The method of producing a sugar liquid according to [1] or [2], wherein the alkaline aqueous medium or the alkaline filtrate is allowed to pass through the cellulose-containing biomass by self-weight filtration in the direction of gravity.

[4] The method of producing a sugar liquid according to any one of [1] to [3], wherein the alkaline aqueous medium and the alkaline filtrate are maintained substantially at the same temperature.

[5] The method of producing a sugar liquid according to any one of [1] to [4], wherein the alkaline aqueous medium and the alkaline filtrate have a temperature of 80°C or higher and 100°C or lower.

[6] The method of producing a sugar liquid according to any one of [1] to [5], wherein the alkaline filtrate includes acetic acid or a salt thereof.

[7] The method of producing a sugar liquid according to any one of [1] to [6], wherein the cellulose-containing biomass is one which has been sifted through a sieve with an aperture of 30 mm or more.

[8] The method of producing a sugar liquid according to any one of [1] to [7], wherein the cellulose-containing biomass is one which has been subjected to a dry grinding treatment.

[9] The method of producing a sugar liquid according to any one of [1] to [8], wherein the cellulose-containing biomass is an herbaceous biomass.

[10] The method of producing a sugar liquid according to any one of [1] to [9], wherein the alkaline aqueous medium and the alkaline filtrate include at least one hydroxide selected from sodium hydroxide and potassium hydroxide.

[11] The method of producing a sugar liquid according to any one of [1] to [10], wherein the alkaline aqueous medium and the alkaline filtrate are alkaline aqueous solutions.

[12] The method of producing a sugar liquid according to any one of [1] to [11], wherein the period of time during which the alkaline filtrate is repeatedly passed through the cellulose-containing biomass is 30 minutes or more and three hours or less.

[13] The method of producing a sugar liquid according to any one of [1] to [12], wherein the alkaline filtrate at the completion of the recirculation filtration step has a pH of 10 or more and 12 or less.

[14] The method of producing a sugar liquid according to any one of [1] to [13], wherein the hydrolysis is carried out using an enzyme.

[15] A pretreatment method of a cellulose-containing biomass, for hydrolyzing the cellulose-containing biomass to obtain a sugar liquid, the method including:

the step of obtaining an alkaline filtrate by allowing an alkaline aqueous medium to pass through a cellulose-containing biomass; and

the recirculation filtration step of allowing the alkaline filtrate to repeatedly pass through the cellulose-containing biomass to produce a cellulose-containing solid component.

[16] A method of producing a cellulose-containing solid component, wherein the cellulose-containing solid component is obtained by the pretreatment method according to [15], from the cellulose-containing biomass.

According to the present invention, it is possible to efficiently obtain a sugar liquid derived from a cellulose-containing biomass, by carrying out a pretreatment in which an alkaline filtrate is repeatedly used. The present invention is advantageous in that it allows for drastically reducing the amount of alkaline substance used in the pretreatment and the reaction time thereof, in the production of a sugar liquid. Further, the present invention is advantageous in that it allows for producing a sugar liquid with a markedly high purity.

DETAILED DESCRIPTION OF THE INVENTION

The method of producing a sugar liquid according to the present invention is characterized in that it includes: the step of obtaining an alkaline filtrate by allowing an alkaline aqueous medium to pass through a cellulose-containing biomass; the recirculation filtration step of allowing the alkaline filtrate to repeatedly pass through the cellulose-containing biomass to produce a cellulose-containing solid component; and the step of hydrolyzing the cellulose-containing solid component to obtain the sugar liquid. It is an unexpected fact that it is possible to drastically reduce the amount of alkaline substance used and the reaction time in the alkaline treatment of the cellulose-containing biomass raw material, and in addition, to efficiently obtain a sugar liquid with a markedly high purity, by carrying out a pretreatment in which the alkaline filtrate, as it is, is repeatedly passed through the cellulose-containing biomass.

In the method according to the present invention, firstly, the cellulose-containing biomass and the alkaline aqueous medium are supplied into a filtration apparatus. In the present invention, the cellulose-containing biomass and the alkaline aqueous medium may be mixed in advance and then supplied into the filtration apparatus, or alternatively, the cellulose-containing biomass and the alkaline aqueous medium may be separately supplied into the filtration apparatus. However, it is preferred that the cellulose-containing biomass be supplied into the filtration apparatus in advance, and then the alkaline aqueous medium be supplied on top of the cellulose-containing biomass.

The cellulose-containing biomass according to the present invention refers to a biological resource containing at least cellulose. Suitable examples of the cellulose-containing biomass include herbaceous biomasses such as bagasse, switchgrass, napier grass, *Erianthus*, corn stover, straw (rice straw and wheat straw), and oil palm empty fruit bunches; woody biomasses such as wood, wood chips, and waste construction materials; and water environment-derived biomasses such as algae and seaweeds; and grain hull biomasses such as corn hulls, wheat hulls, soybean hulls, and rice hulls. However, herbaceous biomasses such as bagasse, rice straw and oil palm empty fruit bunches are more preferably used.

The shape of the cellulose-containing biomass according to the present invention is not particularly limited. However, the cellulose-containing biomass which has been subjected to a grinding treatment is preferred. The means for grinding is not particularly limited, and the grinding treatment can be carried out using a machine commonly used for the coarse grinding of various types of materials, such as, for example, a ball mill, a vibration mill, a cutter mill, a hammer mill, a Wiley mill, or a jet mill. The mechanical grinding as described above may be either a dry grinding or a wet grinding, but a dry grinding is preferred. The biomass may be classified as necessary, after being subjected to the grinding treatment. A preferred range of the grain size of the ground biomass can be selected depending on the aperture size of a sieve through which the cellulose-containing biomass is passed. A preferred range of the aperture size of the sieve through which the cellulose-containing biomass is passed may be, for example, about 8 mm or more, about 8 mm or more and about 20 mm or less, about 20 mm or more, about 20 mm or more and about 50 mm or less, about 30 mm or more, about 30 mm or more and about 50 mm or less, about 50 mm or more, about 50 mm or more and about 70 mm or less, or about 70 mm or more.

Further, the cellulose-containing biomass according to the present invention preferably has a moisture content within the range of, for example, about 3% or more, about 3% or more and about 60% or less, about 5% or more, about 5% or more and about 60% or less, about 5% or more and about 55% or less, or about 5% or more and about 50% or less, but not particularly limited thereto.

The alkaline aqueous medium according to the present invention may be, for example, an alkaline aqueous solution such as an aqueous medium containing ammonia, ammonia water, or a hydroxide. However, the alkaline aqueous medium is preferably an aqueous medium containing at least one hydroxide selected from sodium hydroxide and potassium hydroxide, and more preferably an aqueous solution of sodium hydroxide or an aqueous solution of potassium hydroxide.

The alkali concentration of the alkaline aqueous medium can be calculated based on the content of an alkaline substance(s) (an alkaline solid component(s) such as a hydroxide) contained in the alkaline aqueous medium. The upper limit value of the alkali concentration of the alkaline aqueous medium is preferably about 3, 2, 1.5, 1, 0.7, 0.6, 0.5, 0.4, 0.3 or 0.2% by weight; and the lower limit value thereof is preferably about 0, 0.5, 0.1, 0.2, 0.3, 0.4 or 0.5% by weight, but not particularly limited thereto. The alkaline aqueous medium preferably has an alkali concentration within the range of, for example, about 0.05% by weight or more and about 0.3% by weight or less, about 0.1% by weight or more and about 3% by weight or less, or about 0.1% by weight or more and about 2% by weight or less; more preferably within the range of about from 0.1 to 2% by weight, about 0.25% by weight or more and about 1.5% by weight or less, or about 0.25% by weight or more and about 1.5% by weight or less; and still more preferably within the range of about 0.25% by weight or more and about 1.0% by weight or less.

Further, the lower limit value of the pH of the alkaline aqueous medium is not particularly limited, as long as the pH is within an alkaline range. However, the alkaline aqueous medium has a pH of 7 or more, preferably a pH of 8 or more, more preferably a pH of 9 or more, and still more preferably a pH of 10 or more. The upper limit value of the pH of the alkaline aqueous medium is not particularly limited, as long as the pH is less than 14, and it can be set to a pH of 13.5 or less, from the viewpoint of reducing the amount of alkaline substance used. The alkaline aqueous medium preferably has a pH within the range of, for example, 7 or more and 13.5 or less, or 8 or more and 13.5 or less, more preferably has a pH within the range of 9 or more and 13.5 or less, and still more preferably has a pH within the range of 10 or more and 12 or less.

Acetic acid or a salt thereof may be optionally added to the alkaline aqueous medium. The addition of acetic acid or a salt thereof to the alkaline aqueous medium is preferred in that it allows for improving the reaction efficiency in the present invention. A preferred concentration of acetic acid in the alkaline aqueous medium is, for example, about 0.05% by weight or more and about 5.0% by weight or less, about 0.08% by weight or more and about 3.0% by weight or less, about 0.08% by weight or more and about 2.5% by weight or less, about 0.08% by weight or more and about 2.3% by weight or less, or about 0.1% by weight or more and about 2.0% by weight or less; and more preferably within the range of about 0.08% by weight or more and about 2.3% by weight or less, or about 0.1% by weight or more and about 2.0% by weight or less.

The upper limit value of the temperature of the alkaline aqueous medium is preferably about 110, 100, 95, 90, 80, 75 or 70°C, and the lower limit value thereof is preferably about 35, 40, 50, 60 or 65°C, but not particularly limited thereto. The alkaline aqueous medium preferably has a temperature within the range of, for example, about 35°C or more and about 100°C or less, about 40°C or more and about 100°C or less, about 50°C or more and about 100°C or less, about 60°C or more and about 100°C or less, about 65°C or more and about 100°C or less, or about 80°C or more and about 100°C or less; more preferably has a temperature within the range of about 60°C or more and about 100°C or less, about 65°C or more and about 100°C or less, or about 80°C or more and about 100°C or less; and still more preferably has a temperature within the range of about 65°C or more and about 100°C or less, or about 80°C or more and about 100°C or less.

In the present invention, the weight ratio of the alkaline aqueous medium to the cellulose-containing biomass (in dry weight) is preferably within the range of, for example, from 100:1 to 2:1, from 90:1 to 3:1, from 50:1 to 5:1, from 30:1 to 5:1, from 25:1 to 7:1, from 25:1 to 7:1, from 25:1 to 5:1, or from 20:1 to 5:1, but not particularly limited thereto.

Further, in the present invention, it is also possible to select the ratio of the alkaline substance-containing aqueous medium to the cellulose-containing biomass (in dry weight), using as an index the amount of alkaline substance used (also referred to as an alkaline reaction amount) to be described later in Reference Example 5. A preferred range of the amount of alkaline substance used is, for example, about 20 mg/g or more and about 300 mg/g or less, about 30 mg/g or more and about 200 mg/g or less, about 40 mg/g or more and about 200 mg/g or less, about 45 mg/g or more and about 180 mg/g or less, about 45 mg/g or more and about 150 mg/g or less, about 45 mg/g or more and about 120 mg/g or less, about 50 mg/g or more and about 100 mg/g or less, or about 50 mg/g or more and about 90 mg/g or less; and a more preferred range of the amount of alkaline substance used is about 45 mg/g or more and about 120 mg/g or less, about 50 mg/g or more and about 100 mg/g or less, or about 50 mg/g or more and about 90 mg/g or less.

The filtration apparatus according to the present invention is not particularly limited, as long as it allows for carrying out the present invention. However, it is preferred that the filtration apparatus at least includes: a biomass accommodating portion for accommodating at least the cellulose-containing biomass; a filtering portion for allowing the alkaline aqueous medium to pass through the cellulose-containing biomass; and a filtrate circulating portion for collecting and circulating the alkaline filtrate obtained from the filtering portion.

The shape of the biomass accommodating portion of the filtration apparatus is not particularly limited, and the biomass accommodating portion may be in the shape of a cylinder, a box, a membrane, a plate, a belt (movable type), or the like. It is preferred that the biomass accommodating portion have at least one opening for supplying the cellulose-containing biomass, the alkaline aqueous medium and the alkaline filtrate into the biomass accommodating portion, at an upper surface or a side surface thereof, and be disposed adjacent to the filtering portion. Further, the filtering portion is preferably disposed at a bottom surface of the biomass accommodating portion. In addition, the filtration apparatus according to the present invention preferably has a structure in which the biomass accommodating portion and the filtering portion are integrally formed.

The size of the biomass accommodating portion is not particularly limited, and the biomass accommodating portion preferably has a capacity or an area which allows for accommodating at least the cellulose-containing biomass. For example, in cases where the biomass accommodating portion is in the form of a cylinder or a box, the biomass accommodating portion preferably has a capacity which allows for containing both the cellulose-containing biomass and the alkaline aqueous medium. In cases where the biomass accommodating portion is in the form of a plate, a membrane, slits or a belt, the biomass accommodating portion preferably has a sufficient area so that the cellulose-containing biomass can be disposed on the biomass accommodating portion.

The shape of the filtering portion is not particularly limited, but the filtering portion is preferably in the shape of a plate, a membrane or a belt, since the cellulose-containing biomass is disposed thereon to carry out the filtration. Further, the filtering portion preferably has pores which allow the alkaline aqueous medium to pass therethrough, without allowing the cellulose-containing biomass to pass therethrough. The above described filtering portion can be composed of a microfiltration membrane (MF) or an ultrafiltration membrane (UF).

The average pore diameter of the pores of the filtering portion can be selected as appropriate depending on the grain size of the cellulose-containing biomass. The filtering portion preferably has an average pore diameter within the range of, for example, from 0.001 $\mu$m to 5 mm, from 0.01 $\mu$m to 5 mm, or from 0.1 $\mu$m to 5 mm. The "average pore diameter" as used herein refers to a mean flow pore diameter, as measured by the mean flow-point method, using a porometer (manufactured by Coster Corporation). The shape of the pores of the filtering portion is not particularly limited, and the pores may be, for example, in the shape of cuts extending in one direction, such as slits. The filtering portion preferably has an aperture ratio of 5% or more and 60% or less, and more preferably 10% or more and 40% or less, but not particularly limited thereto. Adjusting the aperture ratio within the above described range is advantageous in that it allows for preventing fine particles of the biomass from clogging to the filtering portion to result in a reduced filtration speed or a failure to hold the biomass on the filtration apparatus. In cases where the filtering portion has pores in the shape of cuts such as slits, the width thereof is preferably from 0.001 $\mu$m to 5 mm, from 0.01 $\mu$m to 5 mm, or from 0.1 $\mu$m to 5 mm, which is the same as the range described above for the average pore diameter.

Materials for the filtering portion are not particularly limited, and examples thereof include: organic materials such as polysulfone, polyethersulfone, chlorinated polyethylene, polypropylene, polyolefin, polyvinyl alcohol, polymethyl methacrylate, polyvinylidene fluoride, polytetrafluorinated ethylene, and polyacrylate; metals such as iron, titanium, aluminum, and stainless steel, and inorganic materials such as ceramics.

The shape of the filtrate circulating portion is not particularly limited, as long as it allows for collecting the alkaline filtrate obtained from the filtering portion to be reused for the filtration. A suitable filtrate circulating portion at least includes a collecting container portion (a bucket or the like), such as, for example, one which is disposed under the filtering portion and which has an opening for collecting the filtrate.

The filtrate circulating portion may be provided fixed to the filtration apparatus, or disposed so as to be transportable or movable. In cases where the filtrate circulating portion is transportable or movable, the filtrate circulating portion can be transported to the vicinity of the biomass accommodating portion after collecting the alkaline filtrate, for example, and the collected alkaline filtrate can be poured into the biomass accommodating portion, as it is, to carry out recirculation filtration.

Further, in cases where the filtrate circulating portion is provided fixed to the filtration apparatus, or not usually transported or moved, the filtrate circulating portion may further include a line portion (a pipe or the like) for circulating the alkaline filtrate from the filtrate circulating portion to the biomass accommodating portion. The line portion preferably includes an injection inlet (such as an opening in the form of a shower nozzle) for injecting the alkaline filtrate into the biomass accommodating portion. Further, it is preferred that the filtrate circulating portion further include a pump which provides a driving force for circulating the alkaline filtrate. In addition, the filtrate circulating portion preferably has a function which allows for maintaining the temperature of, or heating, the filtrate. It is advantageous to use a filtrate circulating portion having such a function of maintaining the temperature of, or heating, the filtrate, since it allows for preventing the reaction from being disturbed due to a temperature drop, particularly in cases where the initial reaction temperature is high. It is more preferred that the filtrate circulating portion of the filtration apparatus according to the present invention be capable of forcibly maintaining or increasing the temperature of the filtrate, by internally sharing vapor or hot water, entirely or partially within the filtrate circulating portion, by means of a jacket system or a trace system.

Materials for the filtrate collecting portion and the biomass accommodating portion are not particularly limited, and the same materials as those described above for the filtering portion can be used, for example.

It possible to use a known circulation-type extraction (filtration) apparatus, as the filtration apparatusaccording to the present invention.

Suitable examples of the filtration apparatus include a belt type filtration apparatus (LM, manufactured by Desmet Ballestra), a basket type filtration apparatus, a rotary type filtration apparatus (Carousel, Rotocell, REFLEX), a Bonot type filtration apparatus, and a screen-filtration type filtration apparatus. More preferred is an in-tank screen filtration apparatus (manufactured by Izumi Food Machinery Co., Ltd.), or a conveyor-type screen filtration apparatus (Model 2 or Model 3; manufactured by Crown Works Company). The use of such a circulation-type extraction (filtration) apparatus is advantageous, since it allows for reducing the cost of industrial equipment, as compared to the use of a conventional pretreatment apparatus using a high-temperature or a high-pressure container.

According to one embodiment of the present invention, it is possible to use a plurality of filtration apparatuses connected in parallel. In such an embodiment, the respective filtration apparatuses can be connected, for example, such that: a first alkaline filtrate discharged from a first filtration apparatus is injected into a second filtration apparatus through a first line portion; a second alkaline filtrate discharged from the second filtration apparatus is injected into a third filtration apparatus through a second line portion; and a third alkaline filtrate discharged from the third filtration apparatus is injected into the first filtration apparatus through a third line portion.

According to another embodiment of the present invention, the filtration apparatus may include one biomass accommodating portion, one filtering portion, and a plurality of filtrate circulating portions. In such an embodiment, for example, the biomass accommodating portion and the filtering portion can be formed integrally, and can be formed in the shape of a belt which is movable and which has pores. Further, the plurality of filtrate circulating portions can be provided under the movable belt. According to such an embodiment, it is possible to carry out a pretreatment reaction by circulating the alkaline filtrate while moving the biomass by the belt, and thus, it is advantageous from the viewpoint of improving the reaction efficiency.

Further, in another preferred embodiment, a filtration apparatus may be used which is capable of bringing the cellulose-containing biomass into facing contact with the alkaline aqueous medium or the alkaline filtrate. Such a filtration apparatus may be provided with, for example, lids which can be opened and closed to the opening and to the contact surface with the filtering portion, so that the biomass accommodating portion can be sealed during the facing contact. Further, the filtration apparatus may further be provided with a pressurizing portion for applying a pressure required during the facing contact. The use of such a filtration apparatus capable of allowing such facing contact is advantageous from the viewpoint of further improving the reaction efficiency.

In the method according to the present invention, an alkaline aqueous medium is allowed to pass through a cellulose-containing biomass, to obtain an alkaline filtrate. According to the present invention, the alkaline aqueous medium is preferably allowed to pass through the cellulose-containing biomass by self-weight filtration in the direction of gravity, which utilizes the weight of the alkaline solution. The above described self-weight filtration is advantageous, since it allows for achieving a moderate passing rate to improve the reaction efficiency, and serves to compact the biomass to realize a uniform reaction. In cases where the liquid is forced to pass through the cellulose-containing biomass using a pump, in particular, the self-weight filtration is especially preferred from the viewpoint of realizing an effective and uniform reaction.

Further, the alkaline filtrate may have a pH within the same range as that of the alkaline aqueous medium, and the alkaline filtrate preferably has a pH within the range of, for example, 7 or more and 12 or less, or 8 or more and 12 or less, more preferably within the range of 9 or more and 12 or less, and still more preferably within the range of 10 or more and 12 or less. The pH of the alkaline filtrate tends to decrease as the reaction proceeds. This is because soluble lignin components function as neutralizers as the alkaline reaction proceeds, and thus, it is possible to measure how far the reaction has proceeded, based on the degree of pH decrease. In particular, the pH range of the alkaline filtrate at the completion of the recirculation filtration (after the reaction) can be adjusted as appropriate by controlling the initial alkali concentration and the like; however, the pH is preferably within the range of, for example, 7 or more and 12.5 or less, or 8 or more and 12.5 or less, more preferably within the range of 9 or more and 12 or less, and still more preferably within the range of 10 or more and 12 or less. Measuring and determining whether the pH of the alkaline filtrate is within the above described range or not, is an effective means to evaluate if the reaction has proceeded to a level sufficient for carrying out a subsequent hydrolysis step. Further, the alkaline filtrate according to the present invention is preferably used as it is in the recirculation filtration, without further adding an alkaline substance thereto. According to the present invention, it is possible to improve the reaction efficiency without further adding an alkaline substance to the alkaline filtrate, and thus it is advantageous in reducing the cost.

Further, it is preferred that the alkaline filtrate substantially maintain the temperature of the alkaline aqueous medium before being filtered. For example, a suitable temperature of the alkaline filtrate may be substantially within the same range as that of the alkaline aqueous medium (within the range of about ±0.5 to 1°C). The alkaline filtrate preferably has a temperature within the range of, for example, about 35°C or more and about 100°C or less, about 40°C or more and about 100°C or less, about 50°C or more and about 100°C or less, about 60°C or more and about 100°C or less, about 65°C or more and about 100°C or less, or about 80°C or more and about 100°C or less; more preferably has a temperature within the range of about 60°C or more and about 100°C or less, about 65°C or more and about 100°C or less, or about 80°C or more and about 100°C or less; and still more preferably has a temperature within the range of about 65°C or more and about 100°C or less, or about 80°C or more and about 100°C or less. The temperature of the alkaline filtrate can be maintained within the above range by installing a known temperature maintenance device or a heating device to the filtration apparatus.

In the method according to the present invention, the alkaline filtrate is repeatedly passed through the cellulose-containing biomass to prepare a cellulose-containing solid component, as described above. In the present invention, a recirculation filtration step in which the alkaline filtrate is repeatedly passed through the cellulose-containing biomass can be carried out using the filtration apparatus described above.

A suitable period of time during which the alkaline filtrate is repeatedly passed through the cellulose-containing biomass is, for example, about 20 minutes or more and about 72 hours or less, about 20 minutes or more and about 48 hours or less, about 20 minutes or more and about 24 hours or less, about 30 minutes or more and about 48 hours or less, about 30 minutes or more and about 24 hours or less, about 30 minutes or more and about 12 hours or less, about 30 minutes or more and about 6 hours or less, or about 30 minutes or more and about 3 hours or less, but not particularly limited thereto.

A suitable number of times for which the alkaline filtrate is repeatedly passed through the cellulose-containing biomass is, for example, at least twice or more, twice or more and 20,000 times or less, twice or more and 10,000 times or less, twice or more and 1,000 times or less, three times or more and 10,000 times or less, three times or more and 1,000 times or less, or three times or more and 100 times or less, but not particularly limited thereto.

The cellulose-containing solid component can be easily separated from the alkaline filtrate by filtration. The resulting cellulose-containing solid component may be subjected to filtration, drying, compression and/or the like, using a known apparatus, before being subjected to a subsequent hydrolysis step. However, it is particularly preferred that the cellulose-containing solid component be subjected to compression. Examples of compression methods include screw pressing, belt pressing and filter pressing. Preferred is screw pressing.

The cellulose-containing solid component preferably has a moisture content within the range of, for example about 3% by weight or more and about 99% by weight or less, about 20% by weight or more and about 99% by weight or less, about 40% by weight or more and about 99% by weight or less, about 50% by weight or more and about 99% by weight or less, or about 50% by weight or more and about 70% by weight or less; more preferably about 50% by weight or more and about 99% by weight or less; and still more preferably about 50% by weight or more and about 70% by weight or less, but not particularly limited thereto. Reducing the moisture content of the cellulose-containing solid component is advantageous in that it allows for improving the purity of the resulting sugar liquid, and improving the reaction efficiency of an enzyme reaction.

In the method according to the present invention, the cellulose-containing solid component is hydrolyzed to obtain a sugar liquid. In the hydrolysis step, it is possible to use a known hydrolysis method, such as, for example, an acid hydrolysis, alkaline hydrolysis, or an enzymatic hydrolysis. However, the cellulose-containing solid component is preferably hydrolyzed with an enzyme, in an aqueous medium. The sugar liquid to be obtained by such an enzymatic hydrolysis step can be obtained, for example, as an aqueous solution containing an oligosaccharide(s) and/or a monosaccharide(s), including glucose, xylose, arabinose, galactose and xylobiose.

The enzyme to be used is not particularly limited as long as it is a cellulose hydrolase or a hemicellulose hydrolase, and may be, for example, a relatively inexpensive and commercially available enzyme. Examples of cellulose enzyme agents which can be used include: *Acremonium* cellulase (manufactured by Meiji Seika Pharma Co., Ltd.), which is an enzyme derived from *Acremonium;* Accellerase DUET (manufactured by Danisco Japan Ltd.), which is an enzyme derived from *Trichoderma;* and Celluclast 1.5L (manufactured by Novozymes A/S). Examples of hemicellulase enzyme agents which can be used include Optimash BG (manufactured by Genencor International, Inc.). The origin of the enzyme is not particularly limited, but enzymes derived from filamentous fungi are more preferred. Enzymes derived from filamentous fungi abundantly contain enzymes for degrading polysaccharides derived from cellulose-containing biomasses, such as, for example, cellulase, hemicellulase and β-glucosidase. Accordingly, the use such a filamentous fungus-derived enzyme is advantageous when carrying out a hydrolysis reaction of a biomass after being subjected to an alkaline treatment.

The enzyme to be used can be selected taking into consideration the properties of the enzyme agent, the composition of a desired product and the like, and it can be used alone or in combination. A suitable amount of the enzyme can also be selected as appropriate, without particular limitation. The amount of the above described enzyme can be set, for example, to 0.01 g or more and 1 g or less, and preferably 0.001 g or more and 0.1 g or less, per 1 g of the raw material substrate used. The temperature, pH and time for carrying out the enzymatic hydrolysis can be selected as appropriate depending on the properties of the enzyme or the combination of the enzymes used. A suitable range of the temperature for carrying out the enzymatic hydrolysis is, for example, within the range of from 30°C or higher and 60°C or lower, and more preferably 35°C or higher and 50°C or lower. The pH for carrying out the enzymatic hydrolysis is, for example, 3 or more and 8 or less, and preferably 4 or more 7 or less. Further, the reaction time is, for example, one hour or more and 48 hours or less, and preferably 6 hours or more and 24 hours or less.

The method of separating a sugar liquid from the hydrolyzate of the cellulose-containing solid component is not particularly limited; however, it is preferred to carry out a membrane treatment after performing solid-liquid separation. Further, the resulting sugar liquid can be optionally subjected to a treatment(s) such as filtration, centrifugation, concentration, and/or drying.

The concentration of glucose, xylose or xylobiose in the sugar liquid according to the present invention is not particularly limited, and it can be adjusted as appropriate by controlling the reaction conditions and the like in the respective steps. A suitable concentration of glucose is, for example, about 5 g/L or more and about 1,000 g/L or less, about 5 g/L or more and about 700 g/L or less, about 5 g/L or more and about 550 g/L or less, or about 10 g/L or more and about 550 g/L or less. A suitable concentration of xylose is, for example, about 1 g/L or to 100 g/L or less, about 1 g/L or more and about 50 g/L or less, or about 1 g/L or more and about 10 g/L or less. Further, a suitable concentration of xylobiose is, for example, about 1 g/L or more up to 100 g/L, about 1 g/L or more and about 50 g/L or less, about 1 g/L or more and about 20 g/L or less, or about 1 g/L or more and about 15 g/L or less.

The method according to the present invention is preferred in that it allows for obtaining a sugar liquid with a high-purity. According to the present invention, it is possible to forcibly extract soluble components of lignin and of other structural units contained in the biomass, by an efficient alkaline treatment, and to effectively prevent them from dissolving into the resulting sugar liquid. The present invention as above described is advantageous in that it allows for obtaining a sugar liquid with a markedly higher purity as compared one obtained using a conventional method. Further, since the present invention allows for obtaining a high-purity sugar liquid, the invention also enables to obtain crystals of glucose, which is hitherto unexpected, by cooling the obtained sugar liquid. For example, when the sugar liquid according to the present invention is left to stand under the conditions of 4°C for 24 hours, the resulting crystals of glucose has a purity of at least 95% or more, more preferably 98% or more, and still more preferably 99% or more. Note, that the above described temperature and time for obtaining the crystals are given for illustration purposes, and that the temperature and the time for obtaining the crystals are not particularly limited. For example, seed crystals may be added in order to obtain sugar crystals having a higher purity, and the temperature may be set to a higher temperature, such as 10°C or the like, so that the crystals are formed over a longer period of time.

The resulting sugar liquid can be used as a fermentation raw material. Further, the application of the sugar liquid according to the present invention is not limited to fermentation raw materials and the like, and the sugar liquid can be used for raw materials for use in the production of sugar alcohols, and the production of high-purity glucose and/or xylose.

EXAMPLES

The present invention will now be described more specifically. However, the present invention is in no way limited by these Examples. Units and measurement methods described in the present specification are in accordance with Japanese Industrial Standard (JIS), unless otherwise specified.

Reference Example 1: Method of Measuring Concentrations of Sugars

The concentrations of sugars contained in the saccharified liquid obtained in each of the Examples and Comparative Examples were analyzed by High performance liquid chromatography (HPLC) under the following conditions, and quantified by comparison with standard samples. The results are shown in Table 1.
Columns: Luna $NH_2$ (manufactured by Phenomenex Inc.)
Mobile phase: ultrapure water; acetonitrile = 25:75
Flow velocity: 0.6 mL/min
Reaction liquid: not used
Detection method: RI (differential refractive index)
Temperature: 30°C

Reference Example 2: Method of Measuring Concentrations of Furan Compounds and Aromatic Compounds

The concentrations of furan compounds (HMF and furfural) and phenolic compounds (such as vanillin) contained in the sugar liquid were analyzed by HPLC under the following conditions, and quantified by comparison with standard samples.
Columns: Synergi HidroRP 4.6 mm $\times$ 250 mm (manufactured by Phenomenex Inc.)
Mobile phase: acetonitrile - 0.1% $H_3PO_4$ (flow velocity: 1.0 mL/min)
Detection method: UV (283 nm)
Temperature: 40°C

Reference Example 3: Method of Measuring Concentrations of Organic Acids

The concentrations of organic acids (acetic acid and formic acid) contained in the sugar liquid were analyzed by HPLC under the following conditions, and quantified by comparison with standard samples.
Columns: Shim-Pack SPR-H and Shim-Pack SCR101H (manufactured by Shimadzu Corporation) connected in series
Mobile phase: 5 mM p-toluenesulfonic acid (flow velocity: 0.8 mL/min)

Reaction liquid: 5 mM p-toluenesulfonic acid, 20 mM BisTris, 0.1 mM EDTA·2Na (flow velocity: 0.8 mL/min)
Detection method: electric conductivity
Temperature: 45°C.

Reference Example 4: Method of Measuring Moisture Content

The moisture content of each of the cellulose-containing biomasses to be used in the following experiments was measured. Using an infrared moisture meter ("FD-720", manufactured by Kett Electric Laboratory), a sample was maintained at a temperature of 120°C, and the moisture content (% by weight; hereinafter, simply indicated as %) of the sample was measured, which is a value obtained from the difference between the stable value after the evaporation and the initial value. The moisture content of each of the raw materials used in the Examples is shown in Table 1. Bagasse, rice straw and oil palm empty fruit bunches are classified as herbaceous biomass.

[Table 1]

| Raw material | Moisture content |
| --- | --- |
| Bagasse | 50% |
| Rice straw | 10% |
| Oil palm empty fruit bunches | 15% |
| Wood chips (cedar) | 5% |

Reference Example 5: Method of Calculating Alkaline Reaction Amount

When calculating the alkaline reaction amount in cases where b (g) of a y (%) aqueous solution of sodium hydroxide is added to a (g) of a cellulose-containing biomass raw material having a moisture content of x (%), for example, to allow a reaction to occur, the alkaline reaction amount (unit: mg/g-dry biomass) is represented by the following Equation 1:

$$[\text{Alkaline reaction amount}] = y \times b \times 1000/ \{(100 - x) \times a\}$$
$$(\text{Equation 1})$$

Example 1: Effect of Using Recirculation Filtration (Reduction in Reaction Time and Reduction in Amount of Alkaline Substance Used)

A cutter mill (Baryonyx BRX-400; manufactured by Nara Machinery Co., Ltd.) was used to grind bagasse. The aperture size of the sieve of the cutter mill was set to 50 mm, and the grinding was carried out at a rotational velocity of 600 rpm, while supplying bagasse at a supply rate of 1,000 kg/hr.

A quantity of 5.0 kg of the resulting ground bagasse (moisture content: 50%) was charged into a multifunctional extraction apparatus (manufactured by Izumi Food Machinery Co., Ltd.), and 45 kg of an aqueous solution of sodium hydroxide having a predetermined concentration (initial temperature: 90°C, pH: around 13) was added thereto, through a spray ball provided at the upper portion of the tank of the multifunctional extraction apparatus. A liquid (alkaline filtrate) obtained by self-weight filtration through a filtration net provided within the tank was repeatedly introduced into the multifunctional extraction apparatus through the spray ball. A heating mechanism was provided between the filtration net and the spray ball provided at the upper portion, and a reaction was carried out for a predetermined period of time, while monitoring the temperature. During the reaction, the temperature of the alkaline filtrate was controlled so as not to be lower than 90°C. An impeller equipped to the above described multifunctional extraction apparatus was not used. The bagasse and the cellulose-containing solid component were placed on the filtration net, and an operation of shaping them with an impeller or the like, or an operation of forming them into a slurry was not carried out. The alkaline filtrate was continuously circulated during the predetermined reaction time. The resulting sample was further filtered with a stainless steel strainer having an aperture of 3 mm (aperture ratio: 30%), and the cellulose-containing solid component remaining on the surface of the strainer was pressed with a hand against the strainer surface, to squeeze out the remaining liquid. The thus obtained solid component had a moisture content of 90%. The pH of the resulting filtrate was also measured, and the results are shown in Table 2.

To the thus obtained solid component, RO water and 35% hydrochloric acid were added to achieve a solid concentration on a dry basis of 5% and a pH of 5.0, to prepare a slurry liquid containing the cellulose-containing solid component. A quantity of 5 mL of Accellerase DUET, which is an enzyme manufactured by Danisco Japan Ltd., was added to 500 mL of the resulting slurry liquid. Then the temperature of the slurry was maintained at 50°C, and a reaction was allowed to proceed for 24 hours, while constantly stirring.

The sugar concentrations in the resulting reaction liquid obtained for each set of reaction conditions were measured by HPLC using the method described in Reference Example 1, and the results (after 6 hours and 24 hours) are shown in Table 2. As a result, it has been found out that the use of the method of Example 1 drastically improves the rate of the enzyme reaction, and provides effects such as, for example, reducing the reaction time and the amount of alkaline substance used which are required to produce the same amount of sugars as compared to using the methods of Comparative Examples 1 and 2 to be described later. In particular, the sugar yields 6 hours after the enzyme reaction were significantly improved as compared to those in Comparative Examples 1 and 2.

[Table 2]

| Reaction conditions | | | | Evaluation results | | | | |
|---|---|---|---|---|---|---|---|---|
| Alkali concentration | Initial pH | Alkaline reaction amount | Alkaline reaction time | Filtrate pH* | 6 Hours after enzyme reaction | | 24 Hours after enzyme reaction | |
| | | | | | Glucose | Xylose | Glucose | Xylose |
| [%] | pH | [mg/g] | [hr] | pH | [g/L] | [g/L] | [g/L] | [g/L] |
| 0.3 | 13.0 | 54 | 2.0 | 11.0 | 16.0 | 8.8 | 17.2 | 9.6 |
| 0.4 | 13.1 | 72 | 2.0 | 10.8 | 17.2 | 9.5 | 19.0 | 11.0 |
| 0.5 | 13.2 | 90 | 2.0 | 10.5 | 18.6 | 10.0 | 20.5 | 12.0 |
| 0.6 | 13.3 | 108 | 2.0 | 10.2 | 18.6 | 10.0 | 20.5 | 12.0 |
| 0.5 | 13.2 | 90 | 0.5 | 11.4 | 15.5 | 8.5 | 17.0 | 9.0 |
| 0.5 | 13.2 | 90 | 1.0 | 10.8 | 17.5 | 9.6 | 19.5 | 11.2 |
| 0.5 | 13.2 | 90 | 1.5 | 10.6 | 18.2 | 9.8 | 20.0 | 11.6 |
| 0.5 | 13.2 | 90 | 3.0 | 10.0 | 18.6 | 10.0 | 20.5 | 12.0 |
| *pH at the completion of reaction | | | | | | | | |

Comparative Example 1: In Cases Where Reaction Is Carried Out Under Stand-Still Conditions (Reaction Time and Amount of Alkaline Substance Used)

A quantity of 0.5 kg of ground bagasse (moisture content: 50%) obtained in Example 1 and 4.5 kg of an aqueous solution of sodium hydroxide having a predetermined concentration were introduced into a stainless steel container with a capacity of 10 L. The contents were heated with a gas stove while stirring, until the internal temperature reached 90°C. Subsequently, the stainless steel container containing the bagasse and the aqueous solution of sodium hydroxide was placed in a convection oven which is in a stable state at 90°C, and then left to stand. At this time, the retention time was taken as the reaction time, and the concentration of sodium hydroxide was varied to prepare a plurality of samples of aqueous sodium hydroxide solutions, as shown in Table 3. Subsequently, each of the resulting samples was filtered through a stainless steel strainer with an aperture of 3 mm, and the cellulose-containing solid component remaining on the upper surface of the strainer was pressed with a hand against the strainer surface, to squeeze out the remaining liquid. The thus obtained cellulose-containing solid component had a moisture content of 90%.

The resulting solid component obtained for each set of reaction conditions was subjected to a neutralization reaction and an enzyme reaction, in the same manner as in Example 1. The results are shown in Table 3.

[Table 3]

| Alkali concentration | Alkaline reaction amount | Alkaline reaction time | 6 Hours after enzyme reaction | | 24 Hours after enzyme reaction | |
|---|---|---|---|---|---|---|
| | | | Glucose | Xylose | Glucose | Xylose |
| [%] | [mg/g] | [hr] | [g/L] | [g/L] | [g/L] | [g/L] |
| 0.3 | 54 | 2.0 | 6.0 | 4.8 | 12.0 | 9.2 |
| 0.4 | 72 | 2.0 | 7.2 | 5.6 | 15.4 | 9.6 |
| 0.5 | 90 | 2.0 | 9.0 | 6.0 | 17.2 | 9.8 |
| 0.6 | 108 | 2.0 | 9.6 | 6.6 | 18.0 | 10.6 |
| 0.5 | 90 | 0.5 | 6.5 | 5.0 | 12.0 | 8.5 |
| 0.5 | 90 | 1.0 | 8.4 | 5.6 | 15.6 | 9.2 |
| 0.5 | 90 | 1.5 | 8.8 | 5.8 | 17.0 | 9.6 |
| 0.5 | 90 | 3.0 | 9.6 | 6.5 | 18.0 | 10.8 |

Comparative Example 2: In Cases Where Reaction is Carried Out with Stirring (Reaction Time and Amount of Alkaline Substance Used)

A quantity of 0.5 kg of ground bagasse (moisture content: 50%) obtained in Example 1 and 4.5 kg of an aqueous solution of sodium hydroxide having a predetermined concentration were introduced into a reaction vessel with a capacity of 8 L. An electrothermal heater-type Jacket was attached to the reaction vessel, and the contents of the vessel were heated while stirring, until the internal temperature reached 90°C. The time point at which the internal temperature reached 90°C was taken as the start of the reaction time, and a stirring reaction was carried out for a predetermined period of time. Further, the resulting sample was filtered through a stainless steel strainer with an aperture of 3 mm, and the cellulose-containing solid component remaining on the upper surface of the strainer was pressed with a hand against the strainer surface, to squeeze out the remaining liquid. The thus obtained cellulose-containing solid component had a moisture content of 90%.
The resulting solid component obtained for each set of reaction conditions was subjected to a neutralization reaction and an enzyme reaction, in the same manner as in Example 1. The results are shown in Table 4.

[Table 4]

| Alkali concentration | Alkaline reaction amount | Alkaline reaction time | 6 Hours after enzyme reaction | | 24 Hours after enzyme reaction | |
|---|---|---|---|---|---|---|
| | | | Glucose | Xylose | Glucose | Xylose |
| [%] | [mg/g] | [hr] | [g/L] | [g/L] | [g/L] | [g/L] |
| 0.3 | 54 | 2.0 | 7.0 | 5.8 | 12.2 | 9.6 |
| 0.4 | 72 | 2.0 | 8.6 | 6.0 | 17.2 | 10.0 |
| 0.5 | 90 | 2.0 | 9.6 | 6.4 | 18.5 | 10.5 |
| 0.6 | 108 | 2.0 | 10.0 | 7.2 | 19.0 | 11.0 |
| 0.5 | 90 | 0.5 | 7.0 | 6.0 | 16.2 | 8.6 |
| 0.5 | 90 | 1.0 | 8.4 | 6.4 | 17.8 | 10.0 |
| 0.5 | 90 | 1.5 | 8.8 | 6.6 | 18.0 | 10.2 |
| 0.5 | 90 | 3.0 | 9.8 | 7.0 | 19.2 | 11.2 |

Example 2: Effect Obtained In Cases Where Acetic Acid Is Added

The reaction conditions were unified to those described in Example 1 in which the concentration of the aqueous solution of sodium hydroxide was set to 0.5%, and the reaction time was set to 2.0 hours. In addition, acetic acid was added to the aqueous solution of sodium hydroxide to prepare samples having predetermined concentrations shown in Table 5, and an examination was carried out. Each of the resulting samples was filtered with a stainless steel strainer having an aperture of 3 mm, in the same manner as in Example 1, and remaining liquid was further squeezed out. The thus obtained cellulose-containing solid component had a moisture content of 90%.

The resulting solid component obtained for each acetic acid concentration was subjected to a neutralization reaction and an enzyme reaction, in the same manner as in Example 1. The results are shown in Table 5. The results revealed that acetic acid in the reaction liquid has contributed to the reaction.

[Table 5]

| Acetic Acid Concentration | 24 Hours after enzyme reaction | |
|---|---|---|
| | Glucose | Xylose |
| [%] | [g/L] | [g/L] |
| Not added (Example 1) | 20.5 | 12.0 |
| 0.1 | 20.5 | 12.0 |
| 0.2 | 21.0 | 12.4 |
| 0.5 | 21.6 | 12.8 |
| 1.0 | 22.0 | 13.0 |
| 2.0 | 22.0 | 13.0 |

Example 3: Examination Regarding Grinding Degree of Bagasse

A cutter mill (Baryonyx BRX-400; manufactured by Nara Machinery Co., Ltd.) was used to grind bagasse, and an examination was carried out. The aperture size of the sieve of the cutter mill was set to 8 mm, 20 mm, 30 mm, 50 mm (Example 1), or 70 mm to carry out an examination. The reaction conditions were unified to those described in Example 1 in which the concentration of the aqueous solution of sodium hydroxide was set to 0.5% and the reaction time was set to 2.0 hours. The resulting sample was filtered with a stainless steel strainer having an aperture of 3 mm in the same manner as in Example 1, and remaining liquid was further squeezed out.

The resulting solid component obtained for each aperture size was subjected to a neutralization reaction and an enzyme reaction, in the same manner as in Example 1. The results are shown in Table 6.

[Table 6]

| Aperture size of sieve of grinder | 6 Hours after enzyme reaction | | 24 Hours after enzyme reaction | |
|---|---|---|---|---|
| | Glucose | Xylose | Glucose | Xylose |
| mm | [g/L] | [g/L] | [g/L] | [g/L] |
| 8 | 14.2 | 8.2 | 16.4 | 9.6 |
| 20 | 16.5 | 9.2 | 18.5 | 10.8 |
| 30 | 18.6 | 10.0 | 20.5 | 12.0 |
| 50(Example 1) | 18.6 | 10.0 | 20.5 | 12.0 |

Example 4: Examination of Other Raw Materials: Rice straw, Oil Palm Empty Fruit Bunches and Wood chips (Cedar)

Rice straw (moisture content: 10%), oil palm empty fruit bunches (moisture content: 15%), and Cedar wood chips (moisture content: 5%) were each ground using a cutter mill under the same conditions as in Example 1, to obtain respective raw material biomasses.

Subsequently, using the same multifunctional extraction apparatus as one used in Example 1, 52 kg of a 0.43% aqueous solution of sodium hydroxide was added to each of the raw material biomasses in amounts shown in Table 7, so as to achieve the alkaline reaction amount of about 90 mg/g-dry biomass, and an examination was carried out. Thereafter, the reaction was carried out in the same manner as in Example 1, under the conditions of 90°C for two hours. Each of the resulting samples was filtered with a stainless steel strainer having an aperture of 3 mm in the same manner as in Example 1, and remaining liquid was further squeezed out. The thus obtained cellulose-containing solid component had a moisture content of 90%.

The resulting solid component obtained for each raw material biomass was subjected to a neutralization reaction and an enzyme reaction, in the same manner as in Example 1. The results are shown in Table 8.

Table 7]

| Raw material biomass | Moisture content | Charged amount | Added amount of alkaline solution | Alkali concentration | Alkaline reaction amount |
|---|---|---|---|---|---|
| Unit | % | kg | kg | % | mg/g |
| Rice straw | 10 | 2.75 | 52 | 0.43 | 90.3 |
| Oil palm empty fruit bunches | 15 | 2.9 | 52 | 0.43 | 90.7 |
| Wood chips (cedar) | 5 | 2.6 | 52 | 0.43 | 90.5 |

[Table 8]

| Raw material | 6 Hours after enzyme reaction | | 24 Hours after enzyme reaction | |
|---|---|---|---|---|
| | Glucose | Xylose | Glucose | Xylose |
| - | [g/L] | [g/L] | [g/L] | [g/L] |
| Rice straw | 17.0 | 11.2 | 19.4 | 13.1 |
| Oil palm empty fruit bunches | 15.4 | 8.1 | 18.2 | 10.0 |
| Wood chips (cedar) | 13.6 | 2.0 | 16.5 | 3.0 |
| Bagasse (Example 1) | 18.6 | 10.0 | 20.5 | 12.0 |

Comparative Example 3

For a comparison with Example 4, an examination example will be described in which a stand-still reaction or a stirring reaction was carried out, using rice straw (moisture content: 10%), oil palm empty fruit bunches (moisture content: 15%), and cedar wood chips (moisture content: 5%). The same examination as that carried out in in Comparative Examples 1 and 2 was carried out using the above described respective raw materials.

In the case of carrying out the stand-still reaction, each of the raw material biomasses obtained in Example 4 and a 0.43% aqueous solution of sodium hydroxide were introduced into a stainless steel container with a capacity of 10 L, according to the amounts shown in Table 9.

In the case of carrying out the stirring reaction, each of the raw material biomasses obtained in Example 4 and a 0.43% aqueous solution of sodium hydroxide were introduced into a reaction vessel with a capacity of 8 L, according to the amounts shown in Table 9.

Each of the resulting samples was filtered in the same manner as in Example 4 and remaining liquid was further squeezed out. The thus obtained cellulose-containing solid component had a moisture content of 90%.

The resulting solid component obtained for each raw material biomass under stand-still or stirring condition was subjected to a neutralization reaction and an enzyme reaction, in the same manner as in Example 1. The reaction results after 6 hours are shown in Table 10.

[Table 9]

| Raw material biomass | Moisture content | Charged amount | Added amount of alkaline solution | Alkali concentration | Alkaline reaction amount |
|---|---|---|---|---|---|
| Unit | % | kg | kg | % | mg/g |
| Rice straw | 10 | 0.275 | 5.2 | 0.43 | 90.3 |
| Oil palm empty fruit bunches | 15 | 0.29 | 5.2 | 0.43 | 90.7 |
| Wood chips (cedar) | 5 | 0.26 | 5.2 | 0.43 | 90.5 |

[Table 10]

| Reaction | Stand-still reaction | | Stirring reaction | |
|---|---|---|---|---|
| Raw material | 6 Hours after enzyme reaction | | 6 Hours after enzyme reaction | |
| | Glucose | Xylose | Glucose | Xylose |
| - | [g/L] | [g/L] | [g/L] | [g/L] |
| Rice straw | 7.6 | 6.0 | 8.0 | 6.8 |
| Oil palm empty fruit bunches | 6.8 | 4.8 | 7.6 | 5.0 |
| Wood chips (cedar) | 5.2 | 0.4 | 6.8 | 0.5 |

Example 5: Examination of Temperature of Alkaline Filtrate

An examination was carried out, varying the conditions of the reaction temperature in Example 1. Specifically, the reaction temperature was varied such that both the initial temperature of the aqueous solution of sodium hydroxide and the temperature during the reaction of the alkaline filtrate were adjusted to 70°C, 75°C, 80°C, 90°C or 95°C. The reaction conditions were unified to those described in Example 1 in which the concentration of the aqueous solution of sodium hydroxide was set to 0.5% and the reaction time was set to 2.0 hours.
Subsequently, the cellulose solid component obtained for each reaction temperature under the same conditions as in Example 1 was subjected to a neutralization reaction and an enzyme reaction, in the same manner as in Example 1. The reaction results after 24 hours are shown in Table 11.

Table 11]

| Reaction temperature | 24 Hours after enzyme reaction | |
|---|---|---|
| | Glucose | Xylose |
| [°C] | [g/L] | [g/L] |
| 70°C | 8.2 | 5.0 |
| 75°C | 8.3 | 5.0 |
| 80°C | 16.5 | 9.0 |
| 90°C (Example 1) | 20.5 | 12.0 |
| 95°C | 21.2 | 12.0 |

Example 6: Examination Using Potassium Hydroxide

A case will be described in which potassium hydroxide was used instead of sodium hydroxide, as an alkaline substance to be used in the Example. The reaction time was set to 2.0 hours, and the comparison between sodium hydroxide and potassium hydroxide was carried out based on the evaluation of the amounts of sugars produced according to the method of Example 1.
The results are shown in Table 12. A comparison of Table 2 (sodium hydroxide) with Table 12 (potassium hydroxide) has revealed that, in the case of using potassium hydroxide as an alkaline substance, an amount of from 1.5 to 2 times the amount of sodium hydroxide, on a weight basis, is required.

[Table 12]

| Reaction conditions | | | | | Evaluation results | | | |
|---|---|---|---|---|---|---|---|---|
| Alkali concentration | Initial pH | Alkaline reaction amount | Alkaline reaction time | Filtrate pH* | 6 Hours after enzyme reaction | | 24 Hours after enzyme reaction | |
| | | | | | Glucose | Xylose | Glucose | Xylose |
| [%] | pH | [mg/g] | [hr] | pH | [g/L] | [g/L] | [g/L] | [g/L] |
| 0.4 | 13.0 | 72 | 2.0 | 10.5 | 14.2 | 7.0 | 15.0 | 8.6 |

(continued)

| Reaction conditions | | | | Evaluation results | | | | |
|---|---|---|---|---|---|---|---|---|
| Alkali concentration | Initial pH | Alkaline reaction amount | Alkaline reaction time | Filtrate pH* | 6 Hours after enzyme reaction | | 24 Hours after enzyme reaction | |
| | | | | | Glucose | Xylose | Glucose | Xylose |
| [%] | pH | [mg/g] | [hr] | pH | [g/L] | [g/L] | [g/L] | [g/L] |
| 0.6 | 13.1 | 108 | 2.0 | 10.8 | 16.4 | 9.0 | 17.5 | 10.0 |
| 0.8 | 13.2 | 144 | 2.0 | 11.2 | 17.6 | 9.8 | 19.2 | 11.0 |
| 1.0 | 13.2 | 180 | 2.0 | 11.8 | 18.6 | 10.0 | 20.5 | 12.0 |
| 1.2 | 13.4 | 216 | 2.0 | 12.0 | 18.6 | 10.0 | 20.5 | 12.0 |
| *pH at the completion of reaction | | | | | | | | |

Example 7: Improvement in Purity of Sugars

To the cellulose solid component obtained in Example 1 under the conditions of an added amount of alkaline substance of 90 mg/g-dry biomass and a reaction time of two hours, RO water and 35% hydrochloric acid were added to achieve a solid concentration on a dry basis of 5% and a pH of 5.0, to prepare a slurry liquid containing the cellulose-containing solid component.

A quantity of 80 mg of *Acremonium* cellulase (manufactured by Meiji Seika Pharma Co., Ltd.) was added to 20 L of the resulting slurry liquid. Then the temperature of the slurry was maintained at 50°C, and a reaction was allowed to proceed for 6 hours, while constantly stirring. Subsequently, the resulting saccharified liquid was centrifuged (at 3,000 G for one minute) to separate the solid component, and a microfiltration membrane, Stericup (pore diameter: 0.22 microns), was used to filter the liquid component from the above described solid component. Then 19 L of the resulting liquid component was filtered (filtration conditions: a liquid circulation rate of 2 L/min and a filtration rate of 5 mL/min) using SEPA-II (a flat membrane filtration testing apparatus, manufactured by GE) and a nanofiltration membrane, NFW (manufactured by Synder Filtration, Inc.) to obtain 18.5 L of a filtrate. Further, the resulting filtrate was subjected to evaporative concentration using an evaporator (manufactured by Tokyo Rikakikai Co., Ltd.), until the concentrate was concentrated to 500 mL. The concentrations of the sugars in the resulting sugar liquid were measured by HPLC according to the method described in Reference Example 1. The results are shown in Table 13.

[Table 13]

| Composition | Glucose | Xylose | Xylobiose |
|---|---|---|---|
| Sugar concentration | 502 g/L | 6 g/L | 10 g/L |

When the sugar liquid obtained in Example 7 was left to stand in a refrigerator controlled to 4°C for 24 hours, the generation of crystals was observed. The resulting crystals were recovered, dissolved in water, and then analyzed by HPLC. As a result, it was found out that the crystals were glucose crystals having a purity of 99% or more.

Regardless of the fact that the glucose concentration was lower as compared to that in Comparative Example 4 to be described later, only the present method succeeded in obtaining glucose crystals. It has been found out that the sugar liquid according to the present invention is advantageous in that it allows for obtaining high-purity glucose crystals which can be used as various kinds of industrial raw materials.

Comparative Example 4: Examination of High-Purity Sugar Liquids Obtained According to Methods of Comparative Examples 1 and 2

Sugar liquids were obtained in the same manner as in Example 7, using the respective cellulose solid components obtained according to the methods of Comparative Example 1 (stand-still reaction) and Comparative Example 2 (stirring reaction) under the conditions of an added amount of alkaline substance of 90 mg/g-dry biomass and a reaction time of two hours. The compositions of the resulting sugar liquids were as shown in Table 14. The resulting sugar liquids were left to stand in a refrigerator controlled to 4°C for seven days, but the generation of glucose crystals such as those obtained in Example 7 was not observed.

[Table 14]

| Composition | Glucose | Xylose | Xylobiose |
|---|---|---|---|
| Method of Comparative Example 1 (stand-still reaction) | 510 g/L | 6 g/L | 10 g/L |
| Method of Comparative Example 2 (stirring reaction) | 512 g/L | 6 g/L | 10 g/L |

Example 8: Examination of Simulated Facing Contact Extraction

A unit was prepared which includes: a bottom surface provided with punched holes having a size of 3 mm; and an acrylic cylinder which is attached by adhesion onto the bottom surface, which is capable of accommodating biomass therein, and which allows for self-weight filtration. The above described unit was configured such that the filtrate obtained through the punched holes can be reintroduced from above the acrylic cylinder to be subjected to refiltration, in a convection oven capable of maintaining a 90°C environment. A reaction was carried out, using the above described system, and using 100 g of bagasse and 900 g of an alkaline solution. The following Method A or Method B was used as the reaction method.

Method A

The above described unit was operated at 90°C for one hour, in the same manner as in Example 1.

Method B

In order to simulate the facing contact between bagasse with an alkaline solution, three systems of the above described units (hereinafter, referred to as Units 1, 2 and 3) were prepared. In Unit 1, the alkaline filtrate obtained from Unit 2 was reacted with ground bagasse for 20 minutes. Next, in Unit 2, the alkaline filtrate obtained from Unit 3 was reacted for 20 minutes, with the cellulose solid component after being treated in Unit 1 for 20 minutes. Next, in Unit 3, the cellulose solid component after being treated in Unit 2 was reacted with a 0.5% aqueous solution of sodium hydroxide for 20 minutes.

The cellulose solid component obtained according to Method A, and the cellulose solid component obtained from Unit 3 in Method B, were each subjected to an enzyme reaction and the analysis of sugars in the same manner as in Example 1.

Further, the alkaline filtrate obtained according to Method A, and the alkaline filtrate obtained after the reaction in Unit 1 in Method B, were each subjected to a concentration analysis according to the method described in Reference Example 2.

The results are shown in Table 15.

It can be seen from Table 15 that the use of Method B resulted in an increase in the amounts of sugars produced, and an increase in the amounts of coumaric acid and ferulic acid produced. The results revealed that the facing contact has served to improve the reaction efficiency.

[Table 15]

| Reaction | Saccharified liquid | | | | Alkaline filtrate | |
|---|---|---|---|---|---|---|
| Method | 6 Hours after enzyme reaction | | 24 Hours after enzyme reaction | | | |
| | Glucose | Xylose | Glucose | Xylose | Coumaric Acid | Ferulic Acid |
| - | [g/L] | [g/L] | [g/L] | [g/L] | [mg/L] | [mg/L] |
| Method A | 8.4 | 5.6 | 15.6 | 9.2 | 1000 | 180 |
| Method B | 9.0 | 6.0 | 17.0 | 9.8 | 1100 | 190 |

Example 9: Compression of Alkaline Treated Biomass

The cellulose solid component obtained in Example 1 for each set of reaction conditions was further filtered with a stainless steel strainer (aperture ratio: 40%) with an aperture of 3 mm, and the cellulose-containing solid component remaining on the upper surface of the strainer was pressed with a hand against the strainer surface, to squeeze out the remaining liquid. The thus obtained solid component having a moisture content of 90% was further compressed, using a screw press for filtering surface (manufactured by Fukoku Kogyo Co., Ltd.) with an aperture of 2 mm, to a moisture content of 60%.

To the resulting solid component, RO water and 35% hydrochloric acid were added to achieve a solid concentration on a dry basis of 5% and a pH of 5.0, to prepare a slurry liquid containing the cellulose-containing solid component, in the same manner as in Example 1. A quantity of 5 mL of Accellerase DUET, which is an enzyme manufactured by Danisco Japan Ltd., was added to 500 mL of the resulting slurry liquid. Then the temperature of the slurry was maintained at 50°C, and a reaction was allowed to proceed for 24 hours, while constantly stirring.

The sugar concentrations in each resulting reaction liquid were measured by HPLC using the method described in Reference Example 1. The results (6 hours and 24 hours after the enzyme reaction) are shown in Table 16. It has been confirmed that a solid component more suitable for an enzyme reaction tends to be obtained as compared to Example 1. In the present Example, an acceleration in the decomposition of hemicellulose has been confirmed, in particular.

[Table 16]

| Reaction conditions | | | | Filtrate pH* | Evaluation results | | | |
|---|---|---|---|---|---|---|---|---|
| Alkali concentration | Initial pH | Alkaline reaction amount | Alkaline reaction time | | 6 Hours after enzyme reaction | | 24 Hours after enzyme reaction | |
| | | | | | Glucose | Xylose | Glucose | Xylose |
| [%] | pH | [mg/g] | [hr] | pH | [g/L] | [g/L] | [g/L] | [g/L] |
| 0.3 | 13.0 | 54 | 2.0 | 11.0 | 17.0 | 9.4 | 18.8 | 10.6 |
| 0.4 | 13.1 | 72 | 2.0 | 10.8 | 18.4 | 10.0 | 20.0 | 11.4 |
| 0.5 | 13.2 | 90 | 2.0 | 10.5 | 19.2 | 11.0 | 20.8 | 12.2 |
| 0.6 | 13.3 | 108 | 2.0 | 10.2 | 19.2 | 11.4 | 20.8 | 12.8 |
| 0.5 | 13.2 | 90 | 0.5 | 11.4 | 16.6 | 9.8 | 18.0 | 11.2 |
| 0.5 | 13.2 | 90 | 1.0 | 10.8 | 18.0 | 10.6 | 19.8 | 11.6 |
| 0.5 | 13.2 | 90 | 1.5 | 10.6 | 18.8 | 10.8 | 20.2 | 12.0 |
| 0.5 | 13.2 | 90 | 3.0 | 10.0 | 19.2 | 11.0 | 20.8 | 12.8 |
| *pH at the completion of reaction | | | | | | | | |

Reference Example 6: Analysis of Difference Due to Alkaline Treatment

To specifically analyze the causes for the difference in the sugar yields between Example 1, Comparative Example 1 and Comparative Example 2, the amounts of produced sugars in the accumulated portions of the respective cellulose-containing solid components obtained according to the respective experimental procedures were measured in detail. Specifically, an alkaline treatment was carried out, first, according to each of the experimental procedures under the conditions of an alkali concentration of 0.5%, an alkaline reaction amount of 90 mg/g, and a reaction time of 2.0 hours. Subsequently, a sample of the cellulose-containing solid component was collected separately from each of "the portion within 1 cm from the upper surface", "the central portion" and "the portion within 1 cm from the bottom surface" of the accumulated portion of the cellulose-containing solid component in each of the multifunctional extraction apparatus used in Example 1, the 10 L-stainless steel container used in Comparative Example 1, and the 8 L-reaction vessel used in Comparative Example 2. The separately collected sample was filtered through a stainless steel strainer with an aperture of 3 mm to obtain a cellulose-containing solid component (moisture content: 90%). The resulting solid component obtained for each of the Example and Comparative Examples was subjected to a neutralization reaction and an enzyme reaction. The results are shown in Table 17.

[Table 17]

| Method | Location of sample collection | Evaluation results | | | |
|---|---|---|---|---|---|
| | | 6 Hours after enzyme reaction | | 24 Hours after enzyme reaction | |
| | | Glucose | Xylose | Glucose | Xylose |
| | | [g/L] | [g/L] | [g/L] | [g/L] |
| Example 1 | Portion within 1 cm from the upper surface | 18.6 | 10.0 | 20.5 | 12.0 |
| | Central portion | 18.6 | 10.0 | 20.5 | 12.0 |
| | Portion within 1 cm from the bottom surface | 18.6 | 10.0 | 20.5 | 12.0 |
| Comparative Example 1 | Portion within 1 cm from the upper surface | 4.0 | 2.8 | 6.0 | 4.0 |
| | Central portion | 10.6 | 5.0 | 16.8 | 10.0 |
| | Portion within 1 cm from the bottom surface | 14.2 | 8.4 | 18.2 | 10.5 |
| Comparative Example 2 | Portion within 1 cm from the upper surface | 3.8 | 2.4 | 6.4 | 4.5 |
| | Central portion | 11.6 | 8.0 | 19.0 | 10.6 |
| | Portion within 1 cm from the bottom surface | 16.2 | 9.6 | 19.8 | 11.6 |

It has been confirmed from the results shown in Table 17 that, in cases where the methods of Comparative Examples 1 and 2 were used, the reactions of the alkaline treatment proceeded sufficiently in the bottom portions of the reaction vessels, but increasingly less sufficiently as it gets closer to the upper surface of the vessels, revealing uneven reactions within the vessels. On the other hand, the method according to Example 1 allowed a uniform alkaline treatment to be carried out within the vessel.

**Claims**

1. A method of producing a sugar liquid, the method comprising:

    the step of obtaining an alkaline filtrate by allowing an alkaline aqueous medium to pass through a cellulose-containing biomass;
    the recirculation filtration step of allowing the alkaline filtrate to repeatedly pass through the cellulose-containing biomass to produce a cellulose-containing solid component; and
    the step of hydrolyzing the cellulose-containing solid component to obtain the sugar liquid.

2. The method of producing a sugar liquid according to claim 1, wherein the step of obtaining the alkaline filtrate comprises supplying the cellulose-containing biomass and the alkaline aqueous medium to a filtration apparatus, and allowing the alkaline aqueous medium to pass through the cellulose-containing biomass using the filtration apparatus.

3. The method of producing a sugar liquid according to claim 1 or 2, wherein the alkaline aqueous medium or the alkaline filtrate is allowed to pass through the cellulose-containing biomass by self-weight filtration in the direction of gravity.

4. The method of producing a sugar liquid according to any one of claims 1 to 3, wherein the alkaline aqueous medium and the alkaline filtrate are maintained substantially at the same temperature.

5. The method of producing a sugar liquid according to any one of claims 1 to 4, wherein the alkaline aqueous medium and the alkaline filtrate have a temperature of 80°C or higher and 100°C or lower.

6. The method of producing a sugar liquid according to any one of claims 1 to 5, wherein the alkaline filtrate comprises acetic acid or a salt thereof.

7. The method of producing a sugar liquid according to any one of claims 1 to 6, wherein the cellulose-containing biomass is one which has been sifted through a sieve with an aperture of 30 mm or more.

8. The method of producing a sugar liquid according to any one of claims 1 to 7, wherein the cellulose-containing biomass is one which has been subjected to a dry grinding treatment.

9. The method of producing a sugar liquid according to any one of claims 1 to 8, wherein the cellulose-containing biomass is an herbaceous biomass.

10. The method of producing a sugar liquid according to any one of claims 1 to 9, wherein the alkaline aqueous medium and the alkaline filtrate comprise at least one hydroxide selected from sodium hydroxide and potassium hydroxide.

11. The method of producing a sugar liquid according to any one of claims 1 to 10, wherein the alkaline aqueous medium and the alkaline filtrate are alkaline aqueous solutions.

12. The method of producing a sugar liquid according to any one of claims 1 to 11, wherein the period of time during which the alkaline filtrate is repeatedly passed through the cellulose-containing biomass is 30 minutes or more and three hours or less.

13. The method of producing a sugar liquid according to any one of claims 1 to 12, wherein the alkaline filtrate at the completion of the recirculation filtration step has a pH of 10 or more and 12 or less.

14. The method of producing a sugar liquid according to any one of claims 1 to 13, wherein the hydrolysis is carried out using an enzyme.

15. A pretreatment method of a cellulose-containing biomass, for hydrolyzing the cellulose-containing biomass to obtain a sugar liquid, the method comprising:

the step of obtaining an alkaline filtrate by allowing an alkaline aqueous medium to pass through a cellulose-containing biomass; and
the recirculation filtration step of allowing the alkaline filtrate to repeatedly pass through the cellulose-containing biomass to produce a cellulose-containing solid component.

16. A method of producing a cellulose-containing solid component, wherein the cellulose-containing solid component is obtained by the pretreatment method according to claim 15, from the cellulose-containing biomass.

## INTERNATIONAL SEARCH REPORT

| International application No. |
| --- |
| PCT/JP2017/012643 |

**A. CLASSIFICATION OF SUBJECT MATTER**
*C12P7/10*(2006.01)i, *C12P19/14*(2006.01)i, *C13K1/02*(2006.01)i

According to International Patent Classification (IPC) or to both national classification and IPC

**B. FIELDS SEARCHED**

Minimum documentation searched (classification system followed by classification symbols)
C12P1/00-41/00, C13K1/00-13/00, C13B5/00-99/00

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI(Thomson Innovation), Japio-GPG/FX

**C. DOCUMENTS CONSIDERED TO BE RELEVANT**

| Category* | Citation of document, with indication, where appropriate, of the relevant passages | Relevant to claim No. |
| --- | --- | --- |
| A | JP 2015-139380 A (Taisei Corp.), 03 August 2015 (03.08.2015), claims; paragraphs [0024], [0025], [0029]; fig. 1 (Family: none) | 1–16 |
| A | JP 2011-083738 A (Oji Paper Co., Ltd.), 28 April 2011 (28.04.2011), claims; paragraphs [0013], [0017], [0019], [0020]; examples; fig. 1 (Family: none) | 1–16 |
| A | JP 2014-023484 A (Taisei Corp.), 06 February 2014 (06.02.2014), claims; paragraphs [0008], [0009], [0018], [0045], [0051]; fig. 1 (Family: none) | 1–16 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 June 2017 (19.06.17) | 27 June 2017 (27.06.17) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (January 2015)

**INTERNATIONAL SEARCH REPORT**

| International application No. |
| --- |
| PCT/JP2017/012643 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2011-152067 A (Oji Paper Co., Ltd.), 11 August 2011 (11.08.2011), claims; paragraphs [0029], [0033], [0051], [0055] (Family: none) | 1-16 |
| A | JP 2011-010597 A (The University of Tokyo), 20 January 2011 (20.01.2011), claims; paragraphs [0030] to [0034]; examples (Family: none) | 1-16 |
| P,A | JP 5982549 B1 (Nippon Steel & Sumikin Engineering Co., Ltd.), 31 August 2016 (31.08.2016), claims & WO 2017/057685 A1 | 1-16 |

Form PCT/ISA/210 (continuation of second sheet) (January 2015)

**REFERENCES CITED IN THE DESCRIPTION**

**Patent documents cited in the description**

- JP 2016066886 A
- JP 2011135861 A
- JP 59192094 A
- JP 2014023484 A
- JP 2013220067 A